Europäisches Patentamt

European Patent Office

Office européen des brevets

(11) Publication number: **0 420 549 A2**

# EUROPEAN PATENT APPLICATION

(21) Application number: **90310428.9**

(51) Int. Cl.5: **A61F 2/14**

(22) Date of filing: **24.09.90**

(30) Priority: **25.09.89 US 411682**

(43) Date of publication of application:
**03.04.91 Bulletin 91/14**

(84) Designated Contracting States:
**BE DE FR GB LU NL**

(71) Applicant: **KINGSTON TECHNOLOGIES, INC.**
**2235-B Route 130**
**Dayton New Jersey 08810(US)**

(72) Inventor: **Stoy, Vladimir A.**
c/o Kingston Technologies, Inc., 2235-B
Route 130
Dayton, New Jersey 08810(US)
Inventor: **Stoy, George P.**
c/o Kingston Technologies, Inc., 2235-B
Route 130
Dayton, New Jersey 08810(US)
Inventor: **Lovy, Alena**
c/o Kingston Technologies, Inc., 2235-B
Route 130
Dayton, New Jersey 08810(US)
Inventor: **Delahanty, Francis T.**
c/o Kingston Technologies, Inc., 2235-B
Route 130
Dayton, New Jersey 08810(US)

(74) Representative: **Ablewhite, Alan James et al**
**MARKS & CLERK 57/60 Lincoln's Inn Fields**
**London WC2A 3LS(GB)**

(54) **Corneal lens implant.**

(57) A corneal lens implant device includes at least two layers of hydrogel with an interface between the layers. The hydrogel layers have at least two different water contents (by weight) such that the difference between these water contents is sufficient to create a convergent or divergent lens at the aforesaid interface. The refractive power of the interface of the implant of the present invention is independent of the outside optical medium so that the high permeability and high refractive power can be achieved within the space constraints of the cornea. In preferred embodiments, hydrogels have a water content of at least 40% and preferably 50% by weight with no greater than 99% by weight and preferably no greater than 95% by weight of water content. Preferred embodiments also include the use of hydrogels having both hydrophilic and hydrophobic polymer phases. In some embodiments, the hydrogen layers form a Fresnel optical surface which may be composed of finite optical elements such as prismatic elements with the slope of each element approximating the local slope of a parent optical surface at the same distance from the optical axis. Thus, some preferred embodiments have finite optical elements with a shape such as triangular, rectangular, hexagonal or concentric rings or spirals.

FIGURE 2

## CORNEAL LENS IMPLANT

### BACKGROUND OF THE INVENTION

#### 1. Field of the Invention

The present invention is directed to corneal lens implants and more particularly corneal lens implants of the intrastromal lens type which contributes its own refractive power and may be implanted via freehand intralamellar pocket dissection.

#### 2. Prior Art Statement

Corneal lens implants are one of the emerging means of correcting the refractive properties of the eye. Such lenses can be roughly divided into two types depending on their refractive function and surgical procedure. One type is designed to change the anterior corneal curvature but causes little refractive change in itself. Such an implantable lens requires lamellar keratectomy or a similar surgical procedure. Another type is an Intrastromal lens (ISL) which contributes its own refractive power and can be implanted via free-hand intralamellar pocket dissection. The latter procedure is much simpler and, therefore, more desirable. The stroma is composed of several hundred strong, parallel, collagenous layers. The individual layers are rather weakly bonded together (compared with the strength of the layers themselves) so that it is relatively easy for a surgeon to detach two adjacent layers inside the stroma and make a pocket or a space there to insert the implant. The ease of the surgery and the relatively minor stress to the eye are the main advantages of such a procedure. The main precondition for a new corrective method is that it should be competitive with more traditional means, such as spectacles or contact lenses.

The present state of the art intrastromal corneal lens implant, however, creates a number of problems leading to many inherent drawbacks. First, the prior art lens creates a barrier to the transport of nutrients and metabolites which are both essential to the health of the cornea. The transport inside of the cornea is diffusive because the cornea cannot contain blood vessels. Glucose and other nutrients must be transported into all corneal layers from inside the eye while oxygen is supplied from the outside environment. The metabolites have to leave the cornea in similar fashion. However, if the flow of the nutrients is interrupted by an impermeable barrier, such as the corneal implants which are currently being developed, it is possible to cause irreversible corneal damage due to a lack of nutrients and metabolites.

Second, intrastromal lens implants contribute refractive changes due to the curvatures of the surfaces of the implants and the refractive properties of the materials used to make the implants. The cornea has a refractive index of about 1.38 corresponding to its water content which is about 78% by weight of the cornea. The major part of its refractive power resides on the anterior corneal surface -air interface (about + 49 D) while only a minor portion is contributed by the cornea's posterior surface - intraocular liquid interface (about - 5.9 D). Therefore, the prior art intrastromal lens implant inserted inside the cornea will contribute to the refractive power proportional to the difference of the refractive indices of the cornea and the lens material. For an appreciable refractive change requirement, the implanted lens should have a refractive index substantially different from that of the cornea. This makes the choice of materials of construction difficult.

Third, the cornea is a relatively thin structure of less than one millimeter in thickness and is not well suited to accommodate a bulky implant. Such an implant would deform both the anterior and posterior corneal surfaces and therefore change the refractive properties of the surface. The change of the anterior corneal curvature will cause a substantial refractive change because of the high value of the refractive index difference between the cornea and air (0.38). Deformation of the posterior surface is less critical because the refractive index difference is much smaller (0.05). The refractive changes due to the corneal deformation are superimposed on the intended refractive change for the implanted lens and are very difficult to control. Further, the pressure on the cornea caused by a bulky implant may cause various physiological problems such as pressure necrosis or long term changes in the corneal structure and shape. Last, a thick and bulky implant presents more of a diffusion barrier than would a thin implant.

The use of prior art hydrogel implants to enhance permeability of aqueous solutes is difficult because relatively high water content is required, i.e. similar to the cornea, yet the refractive index of the hydrogel is primarily dependent on water content and a difference between the water content of the cornea and of the hydrogel is necessary to achieve substantially different refractive indices. A hydrogel lens implanted into the cornea with a similar water content to promote aqueous solute permeability will have inherently low refractive power. This obviously, would not satisfy situations where high refractive powers to create significant changes are necessary. It may be very difficult to compensate for the low refractive index difference by a higher curvature of the lens - cornea interface because a steeper curvature means a thicker, bulkier lens and may additionally create distortion of the corneal surfaces.

In addition to the foregoing, the shape of a negative lens is particularly difficult or unsuitable for the intracorneal implantation because they are inherently thicker at the periphery than at the center. As a result, the negative lenses have a higher average thickness than positive lenses of the same absolute refractive power thus presenting more of a diffusion barrier. Additionally, the intralayer space in the stroma is just not suited to accept an implant of this shape. In addition, it is easier to flatten the posterior rather than the anterior corneal surface so that the refractive change due to the corneal deformation is typically positive (i.e. the natural negative refraction of the posterior corneal surface is diminished) and has to be compensated for by an even stronger negative implant.

In view of the above difficulties in satisfying the various criteria for corneal lens implants, it has been recognized that the requirements are generally contradictory and most corneal implants being tested today are either made from materials with a high refractive index such as polysulfones, which can be made very thin but lack permeability for nutrients, or are made of hydrogels which are much more permeable but which have low refractive indexes and require lamellar keratectomy. Further, the state of the art hydrogel lens implant as used in intro-lamellar pocket dissection only provides positive corrections.

The advantages of the present invention are significant over the prior art because they overcome to a great extent, every one of the problems stated above. Thus, with the present invention corneal lens implant, large and predictable refractive corrections for both positive and negative deficiencies are achieved without substantial corneal deformation and without creating a substantial barrier to nutrient transport.

SUMMARY OF THE INVENTION

The present invention is directed to a corneal lens implant device which includes at least two layers of hydrogel with an optical interface between the layers. The hydrogel layers have at least two different water contents (by weight) such that the difference between these water contents is sufficient to create a convergent or divergent lens at the aforesaid interface. The refractive power of the interface of the implant of the present invention is independent of the outside optical medium so that the high permeability and high refractive power can be achieved within the space constraints of the cornea. In preferred embodiments, the hydrogels have a water content of at least 40% and preferably 50% by weight with no greater than 99% by weight and preferably no greater than 95% by weight of water content. Preferred embodiments also include the use of hydrogels having both hydrophilic and hydrophobic polymer phases. In some embodiments, the hydrogel layers form a Fresnel optical surface which may be composed of finite optical elements such as prismatic elements with the slope of each element approximating the local slope of a parent optical surface at the same distance from the optical axis. Thus, some preferred embodiments have finite optical elements with a shape such as triangular, rectangular, hexagonal or concentric rings or spirals.

BRIEF DESCRIPTION OF THE DRAWINGS

The objects and advantages of the present invention as suggested above and further below become more apparent and will be more fully understood from the following detailed description when taken in conjunction with the accompanying drawings, wherein:

Figure 1 shows a prior art hydrogel type corneal implant;

Figure 2 shows a present invention embodiment involving two layers of hydrogel materials having different water contents;

Figure 3 shows a present invention corneal lens implant device having an interface with the shape of a Fresnel surface with an overall geometrical radius of curvature predefined;

Figure 4 shows a present invention device having three hydrogel layers with the central layer having Fresnel surfaces with two different finite optical elements, in this case one being of a positive refractive power and the other being of a negative refractive power; and,

Figure 5 shows a present invention corneal lens implant device made of three separate hydrogel layers wherein the central hydrogel layer creates two separate interfaces with different Fresnel surfaces.

DETAILED DESCRIPTION OF THE PRESENT INVENTION

As mentioned above, the present invention lens implant device avoids the problems of refractive change limitations as well as the problem of nutrient transport barriers and the problem of corneal deformation. Generally, the present invention achieves these results with an intracorneally implanted lens which has at least two hydrogel layers with different water contents, the interface between the two layers acting as a divergent or convergent optical lens. The refractive power of such an interface is, thus, independent of the outside optical medium so that high permeability as well as high refractive power can be achieved within the space constraints of the cornea. Ideally, the hydrogel layers of the present invention are substantially parallel to one another at the external surfaces of the implant and to the cornea so as to minimize the deformation of the cornea and to minimize stresses which would be abnormal to the natural shape of the cornea. The corneal lens implant of the present invention is preferably shaped in a circular fashion and covers an area with the smallest dimension of about 4 mm. The hydrogels used in the present invention corneal lens implant device have at least about 40% water by weight and preferably have at least about 50% water by weight, in equilibrium, measured under osmotic conditions of the eye at 35° C. The water content in the hydrogels in any layer in the implant of the present invention is lower than about 99% by weight and preferably no lower than about 95% by weight, measured under the same conditions as stated.

The present invention includes at least two adjacent hydrogel layers, and in some embodiments three or more layers, having water contents different from each other by at least 10% by weight and preferably by at least 20% by weight. Further, at least one interface layer between the hydrogel layers forms a lens to obtain the corrective results required for the implant. At least one interface between the hydrogels with different water contents will have an optical radius of curvature different than the radius of at least one of the outside surfaces of the hydrogels so as to create the lens effect. Preferably this interface has the shape of a Fresnel optical surface which has a generally different optical curvature than its apparent geometrical curvature.

In one preferred embodiment the corneal lens implant device of the present invention is made up of a convex - concave layer of hydrogel with a water content of between about 55% and 75% by weight, with an essentially or substantially spherical anterior smooth convex surface of a radius of between 7 and 9 millimeters, and a posterior concave surface in the shape of a Fresnel optical surface. Both convex and concave surfaces are essentially parallel in an average distance of about 0.05 to about 0.25 millimeters, these layers being of circular shape with a diameter of about 5 to about 8 millimeters. A second convex - concave layer hydrogel is included which has a water content of between 85% and 95% of water by weight and has an anterior convex surface in the shape of a Fresnel surface exactly negative to (i.e. matching and complementary to) the concave Fresnel surface of the first layer, and a smooth posterior, essentially or substantially spherical concave surface with a radius of 7 and 9 millimeters. Both the convex and concave surfaces of the second layer are essentially parallel in an average distance of about 0.05 to about 0.25 millimeters. Both of the convex - concave layers are intimately connected by matching the Fresnel surfaces of the posterior surface of the first layer and the anterior surface of the second layer. In this manner, the optical lens for implant of the desired correction is obtained.

The above preferred embodiment device of the present invention has the general shape of a shell with an essentially circular footprint with a diameter of about 5 to about 8 millimeters and of essentially uniform thickness of about 0.05 millimeters to about 0.5 millimeters and a similar radii of anterior and posterior surfaces between about 7 and 9 millimeters. It is composed of two substantially parallel hydrogel layers of substantially different water contents and the internal interface has the shape of a predetermined Fresnel optical surface.

In yet another preferred embodiment of the present invention, the corneal lens implant is composed of three substantially parallel layers of different hydrogels. In this embodiment, the anterior and posterior layers, i.e. the outside layers, have water contents of about 85% and about 95% by weight and the middle hydrogel layer has a water content of between about 50% and 75% by weight. Since there are now three hydrogel layers in this embodiment, the middle layer in contact with the two outer layers thus creates two internal interfaces, one being anterior and one being posterior. It is desirable that at least one of the two interfaces have a Fresnel optical surface.

In another embodiment of the present invention, there is preferably three substantially parallel layers of different hydrogels with the two outside having a water content of about 50% and 75% by weight of water with the middle layer having a water content of between 80% and 99%. Again, in this embodiment, at least one of the interfaces created where the layers contact one another is a Fresnel optical surface.

In another preferred embodiment of the present invention, the corneal lens implant device has at least one Fresnel interface with two inherent focal points between two hydrogel layers of different water contents. Preferably, the Fresnel interface is composed of two types of alternating concentric annular sections each type with a different refractive power. Even more preferable, the diameter of the boundaries between each of the two annular sections is proportional to the square root of the number of that annular section relative to the first central section.

In another preferred design of the present invention corneal lens implant device for bifocal purposes, there are two Fresnel interfaces; one of them having two types of finite optical elements, one type with a positive power up to + 5 D and the other type with a negative power up to about - 5 D, while the other interface is a monofocal Fresnel surface.

The hydrogels used in the present invention device are described in detail below. In general, it has been found that the derivatives of polyacrylic and/or polymethacrylic acid are advantageous. Also, particularly preferred are the covalently crosslinked hydrogels and/or hydrogels with physical crosslinking via crystalline regions containing nitrile groups.

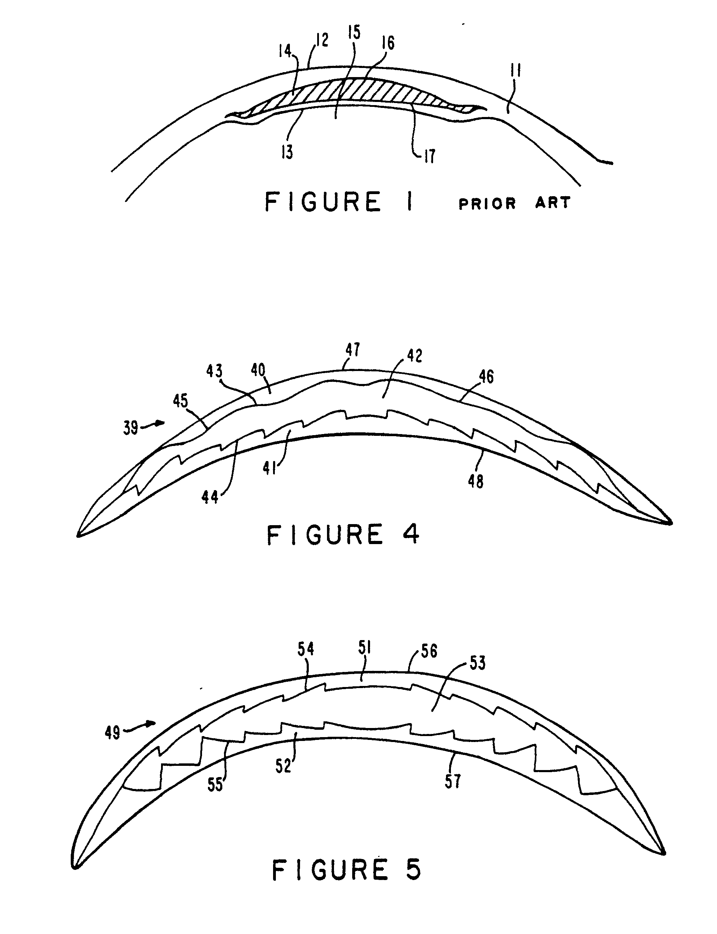

Referring now to Figure 1, there is shown a cornea 11 having an anterior surface 12 and a posterior surface 13 and implanted therein is hydrogel lens 14 having anterior implant surface 16 and posterior implant surface 17 as well as the intracameral liquid 15. Figure 1 represents a prior art arrangement and an analysis has been conducted to show some of the parameters and limitations of this type of implant.

There are several optical interfaces involved, each with a corresponding contribution to the refractive power (all radii are in meters):

1) Interface between corneal anterior surface 12 and air. If the natural radius of curvature is $R_{ac0}$ and the deformed radius is $R_{acd}$, then the change in refractive power is

$D_{ac} = 0.38^*(1/R_{ac0} - 1/R_{acd})$     (1)

2) Interface between anterior surface 16 of implant 14 and cornea 11 with radius $R_{al}$ and refractive power

$D_{al} = (n_h - 1.38)^*(1/R_{al})$     (2)

3) Interface between posterior implant surface 17 and cornea 11 with radius $R_{pl}$ and refractive power

$D_{pl} = (1.38 - n_h)^*(1/R_{pl})$     (3)

4) Interface between posterior corneal surface 13 and intraocular liquid 15. If the natural radius of curvature is $R_{pc0}$ and the deformed radius of curvature is $R_{pcd}$ then the corresponding refractive change is

$D_{pc} = -0.05^*(1/R_{pc0} - 1/R_{pcd})$     (4)

(the usual convention is assumed that radii of convex surfaces are positive and radii of concave surfaces are negative, relative to the direction of the incident beam and the position of the vertex of the surface relative to the center of curvature of the surface). The overall refractive change due to the implant is then approximately (assuming a thin lens and no compression in either tissue or the implant) the sum of refractive changes due to the lens itself and due to the resulting corneal deformation

$D = D_l + D_{cd} = (D_{al} + D_{pl}) + (D_{ac} + D_{pc})$     (5)

$D_{cd}$ could be calculated readily if the anterior and posterior corneal surface would follow the anterior and posterior surface of the implant, because then $R_{acd} = R_{al}$ and $R_{pcd} = R_{pl}$ and D could be calculated from (1) to (5). This is, however, not the case in practice because both the cornea and the hydrogel of the implant are deformable under pressure (changing not only dimension but also water content and refractive index).

In addition, anterior and posterior corneal surfaces have different deformabilities. It is often the case that the anterior corneal surface will stay undeformed, and all of the deformation will be in the implant and on the posterior corneal surface. Moreover, the distribution of deformation between posterior and anterior corneal surfaces will depend on how deep inside the cornea the implant is placed -another parameter difficult to control in practice. Therefore, the corneal deformation is undesirable in itself (causing undesirable physiological effects), but also prevents the prediction of the resulting correction with certainty.

The obviously ideal implant would be such that it would cause minimal corneal deformation, i.e., which would have minimum thickness and

$R_{al} = R_{ac0}$ and $R_{pl} = R_{pc0}$     (6)

It is easy to see, however, that such an "ideal implant" of traditional optical design would cause no corneal deformation but also no refractive correction (of equations 1 to 5)!

The implantable lens according to the present invention has, as mentioned, an internal interface(s) radius of optical curvature $R_{oi}$ between hydrogel layers of different water contents. The hydrogels with different water contents, $Bw_1$ and $Bw_2$, have different refractive indices $n_{h1}$ and $n_{h2}$, respectively. The refractive index of hydrogels is primarily determined by its water content Bw because water -the major component of highly swellable hydrogels - has a very low refractive index (1.335), while the minor polymer component has a similar refractive index (1.49 - 1.52) for most hydrogels.

Hence, the refractive index for most hydrogels can be correlated to their water content by the equation

$n_h = 1.508 - 0.001746^*Bw$     (7)

The refractive power of the internal interface will then be

$D_i = (n_{h1} - n_{h2})^*(1/R_{oi})$     (8)

Because $(n_{h1} - n_{h2})$ can be much higher than $(n_h - n_c)$ in equations (2) and (3), $D_i$ can be much higher than $D_1$ (equation 5). Moreover, the geometry of the internal interface does not directly effect the corneal deformation so that $R_{oi}$ can be selected in a wider range than $R_{al}$ or $R_{pl}$ which have to comply with the corneal shape.

The intrastromal lens can be composed, generally speaking, of N hydrogel layers having (N-I) internal optical interfaces so that the internal optical power is then

$$D_i = D_{i1} + D_{i2} + ... + D_i \, (N\text{-}I) \qquad (9)$$

The equation (5) then becomes

$$D = D_1 + D_{cd} + D_i \qquad (10)$$

Because Di contributes to the refractive power in a major way, the refractive contribution of the outside implant surfaces, $D_1$, can be kept low by meeting condition (6) thereby keeping the refractive contribution of the corneal deformation to a minimum.

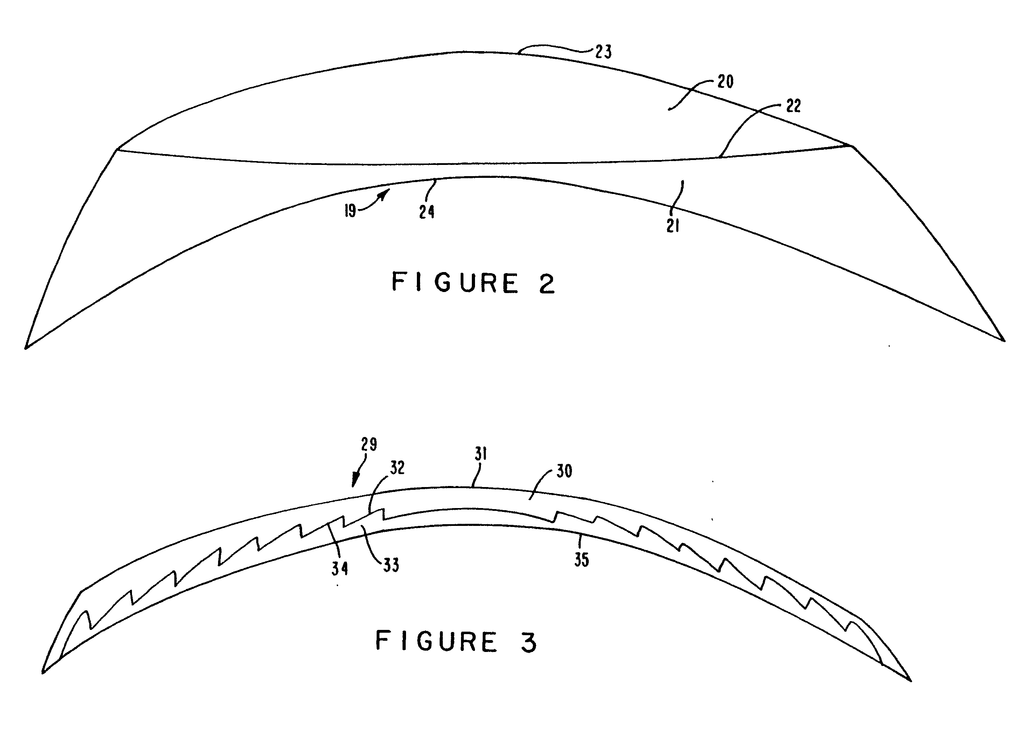

The basic shape of an intrastromal lens according to the present invention is shown in Figure 2 wherein present invention device 19 includes one hydrogel layer 20 having a water content, for example, of 55% and a refractive index of 1.91 and a second hydrogel layer 21 with a water content of 88% and a corresponding refractive index of 1.35. These two layers meet at internal interface 22 which has a radius of a predetermined length so as to create the desired lens results.

Also, as shown in Figure 2, a preferred feature of the present invention involves the use of substantially parallel surfaces for the outer surfaces 23 and 24, respectively, for hydrogel layers 20 and 21. This, as mentioned, diminishes the deformation problem. The corneal lens implant device of Figure 2 has internal interface 22 in the shape of a smooth continuous surface for which the optical radius of curvature equals the geometrical radius of curvature. The relation between the diameter of the lens, its thickness and the radii of the outside surfaces 23 and 24 limits the available shapes and refractive powers of the implant. The implant in Figure 2 has the internal interface in the shape of a smooth, continuous surface for which the optical radius of curvature $R_{oi}$ equals the geometrical radius of curvature $R_{gi}$. There is a rather obvious relation between $R_{gi}$, the diameter of the lens, its thickness and the radii of its outside surfaces $R_{al}$ and $R_{pl}$, which limits the available shapes and refractive powers of the implant. It is preferred, therefore, that the internal interface of the implant has the shape of a Fresnel surface which has an $R_{oi}$ independent of $R_{gi}$. The $R_{oi}$ of a Fresnel surface is proportional to its focal distance rather than related to its overall shape. This allows us to optimize the external shape of the implant for a wide range of refractive powers. The preferred shape of the implant is such that the external shape is that of a thin shell with external radii of curvature, $R_{al}$ and $R_{pl}$, approximating the natural radii of the cornea, $R_{ac0}$ and $R_{pc0}$ respectively. Such an ISL causes a minimal corneal deformation and the inherently unpredictable refractive component $D_{cd}$ is kept to a minimum as well.

As mentioned, Fresnel surfaces are desired in the interface between the hydrogel layers in the present invention implants. Figure 3 shows a present invention embodiment using Fresnel surfaces. Fresnel surfaces may have smooth surfaces with an optical density or refractive index changing from location to location so as to cause bending of an incident beam of light in a similar way as a surface of an ordinary lens. Fresnal surfaces are sometimes also called diffractive or holographic lenses, Fresnel zone plates, phase zone plates, kinoform lenses, thin film lenses or holographic optical elements. More often than not, Fresnel surfaces are surfaces of discontinuous shape where an incident beam bends due to the local surface geometry. One such well known Fresnel surface consists of a prismatic element in a spiral configuration with the slope of the prism approximating the slope of a smooth optical surface at the same distance from the optical axis. Other known Fresnel surfaces are composed of finite optical elements which have the same surface normal at a given distance from the optical axis as a smooth optical surface of similar optical properties ("parent optical surface"). The finite optical elements themselves can have various shapes such as triangular, rectangular, hexagonal, or even random and can be in the shape of annular rings, if desired. One preferred shape for the present invention for finite optical elements would be a combination of essentially circular elements with an Archimedes spiral.

Referring to Figure 3, there is shown corneal lens implant device 29 having hydrogel layer 30 with a water content lower than that of the cornea to which it would be implanted, anterior surface 31 approximating the anterior radius of the cornea and posterior surface 32 in the shape of a Fresnel surface with an overall geometric radius of a preselected curvature and having a predetermined optical radius. Second hydrogel layer 33 has a water content higher than that of the cornea and anterior Fresnel surface 34 is exactly complementary to the surface 32 of layer 30. Thus, these two surfaces brought together create the interface at surfaces 32, 34 which result in a desirable large radius for correction of vision contained within a larger radius arc created by the overall shape of device 29. The optical interface is formed by the surface 33 which has an $R_{oi}$ different from $R_{gi}$. The arrangement in Figure 3 corresponds to the preferred ISL shape of a shell, less than 0.5 mm thick, which has the radii $R_{al}$, $R_{gi}$ and $R_{pl}$ approximately the same and approximately corresponding to the undeformed radii of the cornea. The edge 35 has a wedge shape to allow incorporation of the ISL between stromal layers without causing local stress or local corneal deformation. Because of this, the overall diameter $d_o$ is somewhat larger than the optical diameter $d_d$. Typically, the angle of the edge is between about 15° and 60° and the difference between the overall and optical diameter is typically between about 0.2 and 2 mm.

One of the advantages of utilizing an internal Fresnel Interface in the present invention is that it allows the creation of multifocal, i.e. bifocal or polyfocal, lenses by using finite optical elements of two or more different kinds, each of which is derived from a parent lens with a different refractive power. As a result, the compound Fresnel surface has two or more focal distances from which the eye can select and focus through while disregarding the unfocused elements. In one preferred embodiment of the present invention, a surface with one refractive power is located in the central portion of the lens while the surface which has a different refractive power is located in the periphery of the lens. In this way, the intensity of the focused and unfocused images changes with the aperture of the iris and helps to distinguish near and far objects more clearly. The general concept of having surfaces with two different refractive powers, one located centrally and one located peripherally, is known generally in the optics art and this concept has been used in various optical devices such as contact lenses and intraocular lenses. However, in the present invention, this concept can advantageously be used while still maintaining parallel outer surfaces of the implant and thus minimizing or eliminating the disadvantages cited above.

In accordance with the concept of using different focal distances at different locations along a surface of a lens, the present invention includes a preferred embodiment wherein a bifocal result is obtained which is based on a Fresnel surface with concentric annular finite optical elements with two different sets of focal distances. The finite optical elements may be arranged in an alternating manner, that is, each finite optical element of one set is adjacent to two finite optical elements of the other set. If the outer diameter of each zone is proportional to the square root of the number of the zone as numbered from the center outwardly, then the intensity of the unfocused image in the focal plane of the focused image is diminished by destructive interference while the intensity of the focused image is increased by constructive interference. This improves the bifocal performance and the eye does not need to suppress the unfocused image as in conventional bifocal designs where the bifocal effect is measurable. Thus, in the present invention, the aforesaid bifocal arrangement is used in an embodiment where a Fresnel surface having the concentric annular finite optical elements is an interface between two hydrogel layers.

Figure 4 illustrates a particularly preferred embodiment of the present invention involving a bifocal intrastromal lens. Figure 4 shows implant lens 39 having a hydrogel layer 40 which is a front or anterior layer, a rear or posterior hydrogel 41 and central hydrogel layer 42. First, anterior hydrogel layer 40 has a water content which is higher than that of the cornea, i.e. more than 78% by weight. Posterior hydrogel layer 41 has a water content which is also higher than that of the cornea. Central hydrogel layer 42 has a water content which is lower than that of the cornea. Anterior surface 46 of central hydrogel layer 42 and posterior surface 45 of anterior hydrogel layer 40 form interface 43 which is a Fresnel interface composed of finite optical elements belonging to two different sets, one having a positive refractive power of between about 0 and about 5 Diopters and the other having a negative refractive power in a similar range. Preferably the two sets of finite optical elements alternate with one another as one moves radially from the center so as to achieve the desired bifocal effect. Typically, the difference between the positive and negative sets of finite optical elements will be between about 1 and 5 Diopters and the elements of both sets will alternate in such a way that there are no parasitic, optically inactive surfaces, i.e. as they face the iris, there are no dead areas or areas which are parallel to the optical axis, so that light scattering on the optical discontinuities is minimized. In one preferred embodiment, the width of the finite optical elements may decrease from the center to the periphery. The posterior surface 44 of central hydrogel layer 42 forms a Fresnel surface with a single focal distance so as to form a refractive internal interface with the anterior surface of posterior hydrogel layer 40. The refraction due to the bifocal interface 43 and the monofocal interface 46 superimpose and the optical performance as well as the design flexibility of such a combination appears to be preferable than in the same combination in a single bifocal interface.

In addition to the arrangement described above with respect to Figure 4, it should be noted that many other arrangements may be utilized without exceeding the scope of the present invention. In fact, bifocal effects can be achieved by other means such as creating a polyfocal effect by using aspheric optical surfaces or their combinations. For example, spherical and parabolical interfaces may be combined. Thus, the present invention may accommodate many optical designs which are within the purview of the optical arts.

In the case of compensating for an astigmatism of the eye, one or more of the optical surfaces in a present invention device may be torical or have a toric component. Further, it is also possible to have such a toric component on the outside surface of the implant although it is preferred to have this located on an internal surface to compensate the astigmatism optically rather than by corneal deformation. It is preferred for anti-astigmatic lenses, to have at least two optical interfaces, one having the major toric component and the other having the major spherical component. Combinations of the two surfaces in such a device may cover a large number of requirements and simplify the manufacture of such a device while additionally offering the advantages discussed above.

The difference in water content between the hydrogels in the hydrogel layers of the present invention may be within the ranges cited above and are preferably very high differences. In fact, they should be as high as possible to achieve a high refractive power whether it be negative or positive, while maintaining the internal optical radii as high as possible. At the same time, the water content of any of the hydrogel layers used in the present invention should be as high as possible to maintain high permeability for nutrients and metabolites. Thus, the lowest water content of any of the layers should be at least about 40% and preferably at least about 50% by weight. The highest content in any of the layers is limited by the mechanical properties of available hydrogel materials and is not limited by any other functional requirements. As a practical matter, however, it should now be clear that hydrogels having outside surfaces have mechanical limitations involving structural integrity which are not of concern in the case where internal hydrogel layers are used. Thus, hydrogel layers which are contained or sandwiched between other hydrogel layers may have much higher water contents because they are not exposed to the various factors which could cause their mechanical damage and may be as high as 99% or more by weight of water.

An example of high water content central hydrogel layer devices is illustrated in Figure 5 wherein present invention corneal implant device 49 includes anterior hydrogel layer 51, posterior hydrogel layer 52 and central hydrogel layer 53. Anterior hydrogel layer 51 and posterior hydrogel layer 52 may have lower water content and typically may be of a make up substantially below the water content of the cornea, e.g. about 50% or 55%. However, central hydrogel layer 53 will have very high water content such as 99% and may be more like a polymer solution rather than a gel per se because the outer hydrogel layers will act to sandwich and hold central hydrogel layer 53 in place. Additionally, Fresnel optical surfaces 54 and 55 are shown and may be of the type utilizing concentric bifocal or polyfocal radii. Additionally, as shown, anterior surface 56 and posterior surface 57 are predominantly parallel and would have the approximate curvature of the cornea.

If, to achieve the desired optical results, a Figure 5 type embodiment was required where the water contents of the various layers were reversed, i.e. the outside layers would have a high water content and the central hydrogel layer would have a low water content, to maintain the mechanical integrity of the device, the outside layers should have a water content of about 95% or less by weight. Further, for most of the refractive powers desired, a water content of about 90% is readily achievable and does not impose any serious limit on the optical performance or nutrient transport of the device while assuring mechanical integrity and stability. As indicated, the refractive power is proportional to the difference between the refractive indices of the various hydrogel layers which are in turn proportional to the water content in these layers themselves. Thus, for low refractive power, the difference between hydrogel layers may be small such as a difference of only 5% or so of water content by weight. For many patients, a difference in water content of about 10% to about 15% will satisfy the refractive power requirements. In extreme cases, even differences of up to 40% or more by weight of water between contiguous hydrogel layers is achievable with materials of the present invention.

In preparing the hydrogels for the devices of the present invention, the order of swelling is not of particular importance and those skilled in the art can readily design implantable lenses with outside layers of the highest water content or on the contrary of the lowest water content now that the present invention has been adequately described. Additionally, water content may increase gradually from layer to layer with multiple layers or water content may alternate from high to low or vice versa. Further, the interface between two adjacent hydrogel layers is typically a sharp discontinuity in water content and thus refractive index. It is also possible to form the interface as a continuous gradient between two adjacent layers. Such a gradient is formed as a large number of hydrogel layers with water content and refractive index changes of an infinitesimal difference will be assumed from one layer to the next. In other words, a gradient acts like an infinite number of infinitely thin layers. Thus, the same relations and limitations apply as for design with a finite number of layers with discontinuous interfaces. The advantage of a gradient interface is the suppression of internal reflections of the incident light which can, in some cases, deteriorate the optical performance of the implant. The ability to develop hydrogel gradients, is now within the skill of the artisan developing hydrogel materials.

In preparation of the lens implants of the present invention, once the individual hydrogel layers have been prepared, the contact between the hydrogel layers may be achieved by any number of known means. For example, the hydrogel layers which are to be utilized contiguous to one another may be merely in physical contact with one another or separated by a liquid layer and still perform the refractive function of the present invention. However, in preferred embodiments, the hydrogel layers are mutually interconnected by strong physical forces such as by adhesion or by covalent bonding. In a most preferred embodiment, the covalent interconnection may be achieved by covalent crosslinking of two hydrogel layers by known techniques such as copolymerization of polyfunctional monomers, ionizing irradiation, crosslinking pendant hydrogel groups with aldehydes, diepoxides or diisocyanates or the like.

One of the advantages of the present invention implant design is that it can be designed simultaneously with high refractive power, high permeability, small thickness and large optical diameter. An optical diameter of about 4 millimeters is deemed the minimum to provide the desired function. It is advantageous, however, that the optical radius be large enough to cover the pupil even under poor light conditions. For this reason, an optical diameter of about 6 millimeters appears to be optimum. Diameters larger than about 8 millimeters would require a large incision during surgery while the improvement in optical performance would be marginal. For these reasons, the optical diameter may advantageously be between about 4 and 8 millimeters and preferably between about 5 and 7 millimeters. The overall diameter of the implant should be slightly larger than the optical diameter to allow for a thin, wedgelike edge which would fit readily between stromal layers without causing local stress or local corneal deformation. The overall diameter should be larger than the optical diameter by about 0.2 to 2 millimeters. The overall average thickness of the lens should not exceed about 0.5 millimeters to avoid corneal deformation and action as a barrier to nutrient transport. The thickness should be as small as possible, with a current practical limit being about 0.025 to 0.075 millimeters. The preferred thickness is between this lower limit and about 0.15 millimeters. The overall shape or footprint of the lens is circular or partly circular, such as a truncated circle. The latter shape is preferred for implants with toric optics which require appropriate orientation in the cornea. Various other footprints are also possible, such as elliptical, circular with asymmetric protrusions (ears), etc. should one find such shapes desirable to stabilize the lens' position or facilitate the surgical procedure.

There are numerous hydrogels which can meet the requirements of the hydrogel layer, based on hydrophilic acrylates and methacrylates, acrylamides and methacrylamides, hydrophilic polyesters, vinyl copolymers, etc. There is a broader selection in the area of hydrogels with low water content which have been thoroughly developed and tested as materials for contact lenses, intraocular lenses and surgical implants. The preferred hydrogels are based on hydrophilic derivatives of polyacrylic and polymethacrylic acid which have a long history of hydrolytic and enzymatic stability. The best mechanical properties at very high water contents have typically belonged to hydrogels with two polymer phases, one being hydrophilic and the other hydrophobic, such as multiblock copolymers of acrylonitrile, segmented hydrophilic polyurethanes, etc. The hydrophobic phase forms physical crosslinking which provides the hydrogel with improved strength and resistance to fracture. The covalent crosslinking, on the other hand, provides improved thermal and mechanical stability to the hydrogels, which is an important consideration for the long term implants. The covalently crosslinked hydrogels are preferred materials for this reason. The particularly preferred hydrogels useful in the lens of the present invention are those having both a physical and chemical network because such a structure combines high stability and good mechanical properties at high water content. Examples of such hydrogels are covalently crosslinked hydrogels derived from block copolymers of polyacrylonitrile. Various hydrogel systems and compositions are suitable for the invention and very well known to those skilled in the art. It is not the intention to limit the scope of the invention to any particular chemical composition of the hydrogel as long as the hydrogels meet the physical requirements described in this invention. The lens according to the invention is implantable into the cornea by procedures mentioned herein. Such implantation may be performed into any depth of the cornea, including implantation into a very shallow depth or implantation into the posterior or anterior corneal surface. The present invention describes the implantable lens device and is not limited to any particular surgical procedure or technique.

Obviously, numerous modifications and variations of the present invention are possible in light of the above teachings. It is therefore understood that within the scope of the appended claims, the invention may be practiced otherwise than as specifically described herein.

## Claims

1. A corneal lens implant device comprising: at least two layers of hydrogel and an interface between said layers, said layers of hydrogel having at least two different water contents by weight, the difference between the water contents being sufficient to create a convergent or divergent lens at said interface.

2. The device of claim 1 wherein said hydrogels have a water content of at least 40% by weight.

3. The device of claim 2 wherein said hydrogels have a water content of at least 50% by weight.

4. The device of any of claims 1 to 3 wherein said hydrogels have a water content of no greater than about 99% by weight.

5. The device of claim 4 wherein said hydrogels have a water content of no greater than about 95% by weight.

6. The device of any of claims 1 to 5 wherein the difference between the water contents of said hydrogels is at least about 10% by weight.

7. The device of claim 6 wherein the difference between the water contents of said hydrogels is at least about 20% by weight.

8. The device of any of claims 1 to 7 wherein at least one of said hydrogels is a hydrophillic derivative of polyacrylic acid or polymethacrylic acid.

9. The device of any of claims 1 to 8 wherein at least one of said hydrogels is covalently cross linked.

10. The device of any of claims 1 to 9 wherein at least one of said hydrogels has both hydrophobic and hydrophilic polymer phases.

11. The device of claim 10 wherein said hydrophobic polymer phase contains crystalline polyacrylonitrile domains.

12. The device of any of claims 1 to 11 wherein said interface between two hydrogel layers has the form of a Fresnel optical surface.

13. The device of claim 12 wherein said Fresnel optical surface is composed of Finite Optical Elements.

14. The device of claim 13 wherein said Finite Optical Elements are prismatic elements with the slope of each element approximating the local slope of a parent optical surface at the same distance from the optical axis.

15. The device of claim 13 wherein said Finite Optical Elements have a surface normal on the major portion of the surface the same as the normal to the parent optical surface at the same distance from the optical axis.

16. The device of claim 13 wherein said Finite Optical Elements have a shape selected from triangular, rectangular, hexagonal, concentric rings and spirals.

17. The device of any of claims 13 to 16 wherein said Finite Optical Elements are of at least two different kinds.

18. The device of claim 17 wherein one Finite Optical Element is of a substantially circular shape and located in the center of the lens and the other Finite Optical Element has either a spiral shape or the shape of concentric rings.

19. The device of claim 17 wherein one kind of Finite Optical Element has one refractive power and the other kind of Finite Optical Element has different refractive power thus forming an optical surface with two different focal points.

20. The device of claim 19 wherein Finite Optical Elements of two different kinds are arranged as alternating concentric rings.

21. The device of claim 20 wherein the width of the rings decreases from the center to the periphery of the lens.

22. The device of any of claims 17 to 21 wherein one Finite Optical Element has a negative refractive power.

23. The device of any of claims 1 to 22 wherein there are two hydrogel layers and wherein one of the two hydrogels has a water content between 45% and 70% by weight and the other hydrogel has a water content between 85% and 95% by weight.

24. The device of any of claims 1 to 22 wherein there are three hydrogel layers and two internal refractive surfaces and wherein one internal refractive interface is composed of two kinds of Finite Optical Elements, one kind with positive and one kind with negative refractive power, and the other internal refractive interface is composed of elements with either negative or positive refractive power only.

25. The device of any of claims 1 to 22 and 24 wherein there are three hydrogel layers and two internal refractive interfaces and wherein one or both of said internal refractive interfaces has/have a toric optical element.

26. The device of any of claims 1 to 25 wherein said interface is a gradient interface with water content changing gradually from one layer to the next.

27. The device of any of claims 1 to 26 wherein the outer surfaces have simialr radii of curvature so that the lens has the shape of a spherical shell.

28. The device of claim 27 wherein the thickness of said spherical shell is smaller than 0. 5 mm.

29. The device of any of claims 1 to 28 wherein the lens has an edge in the shape of a wedge with an angle between about 15 and 60°.

30. The device of claim 29 wherein the wedge is between 0.2 and 1 mm wide.

31. The device of any of the preceding claims wherein the projection of the lens onto a plane perpendicular to the optical axis has the smallest dimension larger than 4 mm and the largest dimension smaller than 9 mm.

32. The device of claim 31 wherein said projection is substantially circular, is substantially elliptical or is a truncated circle.

FIGURE I    PRIOR ART

FIGURE 4

FIGURE 5

FIGURE 3

FIGURE 2